Europäisches Patentamt

European Patent Office

Office européen des brevets

(19)

(11) Publication number: 0 335 293
A2

## EUROPEAN PATENT APPLICATION

(21) Application number: 89105355.5

(22) Date of filing: 25.03.89

(51) Int. Cl.4: G01N 33/564 , G01N 33/567

(30) Priority: 30.03.88 JP 74468/88

(43) Date of publication of application:
04.10.89 Bulletin 89/40

(84) Designated Contracting States:
AT BE CH DE ES FR GB GR IT LI LU NL SE

(71) Applicant: HOECHST JAPAN LIMITED
10-16, 8-chome, Akasaka, Minato-ku
Tokyo(JP)

(72) Inventor: Jitsukawa, Tomofumi
Mezon-Yamataka C-201 Minamiohtsuka,
787-1
Kawagoe-shi Saitama-ken(JP)

(74) Representative: Becker, Heinrich Karl
Engelbert, Dr. et al
HOECHST AKTIENGESELLSCHAFT Central
Patent Department P.O. Box 80 03 20
D-6230 Frankfurt am Main 80(DE)

(54) A method of preparing an analytical element for the determination of anti-nuclear antibodies.

(57) A device for measuring an anti-nuclear antibody is prepared by contacting a suspension of cell nuclei with a support to absorb the cell nuclei on the support, separating the support from said suspension and treating the surface of the support with a 50-90% acetone solution to fix the cell nuclei on the support. By using the device, anti-nuclear antibody in blood or body fluid can easily and simply be measured with high accuracy so that the device is usable for the diagnosis of autoimmune disease, particularly in the primary screening. The device can be manufactured at low cost and with simple procedures.

**A method of preparing an analytical element for the determination of anti-nuclear antibodies**

Background of the Invention

1. Field of the invention

The present invention provides a method of preparing device for measuring anti-nuclear antibody and a device prepared thereby. By the use of the device according to the invention, anti-nuclear antibody in blood or body fluid can easily and simply be measured with high accuracy so that the device is usable for the diagnosis of autoimmune diseases, particularly in primary screening. As the invention can provide the assay at low cost and with simple procedure, measurement of anti-nuclear antibody which has heretofore been performed only in special cases becomes of wide use in general health inspection; and this enables diagnosis and therapy of autoimmune diseases carried out at earlier stages.

2. Prior art

There is often detected anti-nuclear antibody at high concentration in blood of patients with autoimmune diseases such as systemic lupus erythematosus (SLE), progressive systemic sclerosis (PSS), rheumatoid arthritis (RA), Sjoegren syndrome (SJS), dermatomyositis (DM), polymyositis (PM) and mixed connective tissue disease (MCTD).

More than 20 different anti-nuclear antibodies are known against a variety of antigens present in the cell nucleus such as anti-ds- or ss-DNA antibody, antihistone antibody, anti-nonhistone nuclear protein antibody, anticentromere antibody, anti-U1-RNP antibody and anti-Sm antibody. As is the case with anti-ds- or ss-DNA antibody on the presence of which diagnosis for SLE is based, each anti-nuclear antibody is known for the relationship with various autoimmune diseases, and detection of such antibody is helpful to the diagnosis of autoimmune disease.

The standard method most commonly employed for detecting the anti-nuclear antibody is fluorescent antibody technique [a review; "Rinsho Kensa" (Clinical Tests), vol. 30, No. 7, 1986, pp. 684-728]. In addition, such methods as double immunodiffusion, RIA, ELISA and hemagglutination are mentioned. In the fluorescent antibody technique a tissue slice or cells of an established strain fixed on a glass slide are used as antigen material. The presence or absence of anti-nuclear antibody in a specimen such as blood are observed using fluorescence microscope technique to determine the straining pattern and thus the nature of the anti-nuclear antibody. Utility of the technique is widely accepted in the diagnosis of autoimmune diseases because relationship between the fluorescent staining pattern and the anti-nuclear antibody has extensively been investigated. The fluorescent method, however, is disadvantageous in that precise judgement depends very much on the operator, as the evaluation can only be done visually, fluorescent dyes are rather unstable and difficult to handle and sensitivity of the fluorescence microscope and microscopic examination conditions influence the judgement.

Then, ELISA (enzyme linked immunosorbent assay) has been developed, by which variations between individuals are smaller, operations of which are easy and simple and which produces quantitative results. ELISA for the anti-nuclear antibody is broadly divided into two classes. One of the ELISA methods uses purified antigens separated from the cell nucleus such as a variety of DNA, RNA histone protein and nonhistone protein, purified and fixed on a plastic support by which the anti-nuclear antibody can be identified. As it is cumbersome to conduct the ELISA for all nuclear antigens, the ELISA is used mainly for secondary screening together with or independently of the fluorescent method.

Mainly for primary screening for rapidly detecting the presence of a wide variety of anti-nuclear antibodies, an ELISA technique has been developed in which an extractable nuclear antigen (ENA) is fixed on a plastic support (R. Warlow et al. "Diagnostic Immunology", vol. 2, pp. 154-160, 1984). In this technique a nuclear antigen of a tissue such as the liver or of a cell strain which has been solubilized or extracted with an acid is utilized. However, the technique has often failed to detect and anti-nuclear antibody against a nuclear antigen which is insoluble or unextractable with an acid. Judgement according to the technique, therefore is not consistent with judgement given by the standard fluorescent antibody technique in some cases.

Further in 1987, a patent application by V. L. Lipp et al. entitled "method and apparatus for detecting anti-nuclear antibody" has been laid open to the public (Japanese Patent Laid Open Sho 62-32363) wherein the nucleus is supported on a solid support by drying so that insoluble nuclear antigen is detectable.

The technique, however, requires complicated operation in which not only the nucleus but also nuclear antigen (NS) and ENA produced by treatment with a surfactant and subsequently by ultrasonic wave are prepared and the combined three are supported on a solid support. There is used in the technique undiluted serum as test material which results in lower sensitivity. Direct use of undiluted serum is also apt to detect background reaction owing to a large amount of nonspecific antibody which contaminates the test material. Moreover, in the specification of the above-cited laid open publication no example of the actual measurement for such a test material as blood from patients with autoimmune diseases is given. Therefore, further improvement is deemed necessary for practical use.

Summary of the Invention

It is an object of the invention to provide a method which comprises preparing a device for ELISA to measure anti-nuclear antibody by simple procedures. Another object is to provide a device for screening anti-nuclear antibody with high accuracy.

Detailed Description of the Invention

According to the present invention, the cell nucleus is fixed to a support by directly contacting a suspension of cell nuclei with the support to adsorb the cell nuclei on the support, separating the support from the suspension and treating it with a 50-90% acetone solution to fix the cell nuclei on the support.

Cell nuclei used in the present invention are from cell strains of higher animals, particularly of human origin. As especially preferred cell strains are mentioned HEp-2, Wil-2, A-549, PC-13 and ZR-751. The preparation of the cell nuclei suspension is described below.

Cells are cultivated by methods known to the person skilled in the art, collected, washed with an isotonic buffer solution and then suspended in a hypotonic buffer solution of reduced salt concentration to swell them to such an extent that the cells are not broken. The swollen cells are greatly broken by simple homogenizing operation so that cell nuclei are produced relatively intact.

As isotonic buffer solutions used herein are mentioned Tris buffer solution, phosphate buffer solution and the like. Suitable hypotonic buffer solutions are those which have a salt concentration in the range of 1/2 to 1/15 that of the isotonic solution. It is desirable to break cells to an extent of 90% or more by homogenizing operation. In addition to the homogenizing operation, ultrasonic treatment is also feasible for breaking cells.

The solution of broken cells thus obtained are centrifuged to collect the nucleus fraction. The nucleus fraction thus isolated is resuspended in the above-mentioned hypotonic solution to prepare a suspension of the nucleus fraction. Concentration of the nucleus fraction in the suspension is desirably $2 \times 10^5$/ml or more in terms of the cell nucleus.

The suspension of the cell nucleus fraction was directly contacted with a support, preferably a plastic support to adsorb the nucleus reaction on the support. As the plastic support are used those of polystyrene, surface-treated polystyrene, polyvinyls and the like. Polystyrene plate with 96 wells for ELISA is commercially available and is conveniently used as it is. The support may be in any shape such as test tube or plate. Fixation of the nucleus fraction suspension to the plastic support can be effected by allowing to stand at room temperature for 30-120 minutes. The temperature and time may be varied within an appropriate range. If an ELISA plate with wells is employed, the nucleus fraction suspension may be divided into the wells followed by standing for a necessary period of time.

Then, the plastic support and the nucleus fraction suspension are separated, and the plastic support is treated with a 50-90% acetone solution to fix the nucleus fraction on it. If an ELISA plate with wells is employed, the nucleus fraction suspension is removed by an appropriate means such as suction, and then a 50-90% acetone solution in the same amount as that of the nucleus fraction suspension is divided into the wells. Usually, allowing to stand at room temperature for 10 min. or longer results in fixing of the cell nuclei on the plastic support. The cell nuclei are not peeled off by washing. If washing is made after removal of the nucleus fraction suspension from the wells, the cell nuclei will be washed out; therefore, washing is usually undesirable. However, the washing may be made if number of the cell nuclei to be carried on the support is to be controlled. It is desirable to employ a 85% solution as the acetone solution. Use of an acetone solution in a concentration of 90% or higher is undesirable because plastic support will be damaged by the acetone. Whereas the acetone is preferably in aqueous solution, a part of the water may be replaced by a polar solvent such as ethyl alcohol. Temperature and time for the treatment may be varied with in an appropriate range. The acetone treatment according to the invention not only results in fixing of cell nuclei on the support but also facilitates approach of anti-nuclear antibody to nuclear antigen inside the nucleus by dissolving phospholipid membrane of the cell nucleus. This greatly contributes to improving accuracy in the assay.

Such treatment of the cell nucleus-carrying plastic ELISA plate prepared in the invention enable measurement of anti-nuclear antibody in which 1:100-diluted serum is satisfactorily used, with good sensitivity as well as with low background owing to nonspecific antibodies co-existing in the undiluted test material. When such plate is employed, the presence or absence of anti-nuclear antibody in patients with autoimmune diseases can be screened with high accuracy without application of nuclear antigen (NS) and ENA as carried out in Japanese Patent Laid Open Sho 62-32363. Nevertheless, ENA or the like may also be carried on the support in order to facilitate the detection. In this case, ENA is adsorbed on the plate prepared according to the method of the invention by contracting it with ENA.

Detection of anti-nuclear antibody using the nucleus-carrying plastic support prepared in the present invention can be carried out in the same way as with the ELISA method in which extracted nuclear antigen is carried on a plastic support.

As a matter of fact, serum to be tested is reacted by adding it to plastic wells on which cell nuclei are carried according to the invention, removing unreacted serum by washing, then, for example, rabbit anti-human immunoglobulin antibody or protein G labelled with horse radish peroxidase (HRPO) is reacted to form bond with the anti-nuclear antibody bonded with cell nuclei, and after washing, the HRPO is color-developed with tetramethylbenzidine (TMB) color developer.

According to the invention, there is produced a plastic plate for detecting anti-nuclear antibody with high accuracy and by simple procedures. The plastic plate thus produced can be stored without decreasing its effectiveness over one year or longer if maintained at low temperature under low humidity.

The invention will be described in detail below with reference to an example and a test example.

Example

(Culture of the cell)

As a source for preparing the nucleus fractions of eukaryotic cells, HEp-2 cell strain of human-laryngeal cell-epithelioma origin (ATCC CCL23) was purchased from ATCC (American Type Culture Collection). The purchased HEp-2 cell is cultured in 10% FBS-RPMI 1640 medium, and the culture is promptly freeze-stored. For actually preparing the cell nucleus, the freeze-stored cell is defreezed and grown for about a week, and $1 \times 10^8$ cells are collected. To peel the cells off the culture flask, 0.02% EDTA-phosphate buffer solution (PBS) is employed (prepared by dissolving 14.5 g of $Na_2HPO_3.12H_2O$; 1.0 g of $KH_2PO_4$; 40.0 g of NaCl and 1.0 g of KCl in distilled water, adjusting pH and volume of the solution to 7.2 and 5 liters, respectively, additionally dissolving $EDTA.2Na.2H_2O$ and subjecting the solution to autoclave sterilization).

(Extraction and fixation of the cell nucleus)

In Tris buffer solution (TBS) (0.01M Tris-HCl, 0.15M NaCl, pH 7.4) were suspended $1 \times 10^8$ HEp-2 cells. Washing was made with two 50-ml portions of TBS, and the cells were collected as precipitates by centrifugal separation carried out under $500 \times g$ for 5 min. The cells were suspended in 20 ml of a hypotonic buffer solution (0.01M Tris-HCl, 0.01M NaCl, 1 mM $MgCl_2$, pH 7.4), and the suspension was allowed to stand on ice for 15 min. The cells were crushed in a Downes homogenizer. After confirming by microscope crushing of 90% or more, preferably 95% or more of the cell membrane, the crushed cells were again subjected to centrifugal separation under $2,000 \times g$ for 10 min. Nucleus fractions produced as the residue was suspended in the same hypotonic buffer solution as above in such a way as providing a concentration of $2 \times 10^5$/ml in terms of the cell nucleus, and 50 $\mu$l of the suspension was poured to each of the wells of a plastic microplate (commercially available, for example, from Nunc). The plastic plate was allowed to stand at room temperature for 30 min. Supernatant of the poured suspension was then removed completely by suction by means of an ELISA processor or a similar device (for example, Model BEP-II of Behring). Then, to each of the wells was poured 50 $\mu$l of 85% aqueous solution of acetone followed by standing at room temperature for 10 min. The acetone solution was removed by suction, and the plastic plate was dried in a drier. The cell nuclei-supporting plastic plate thus prepared, when packed in a packaging material such as Suran Wrap, could be stored at 4°C for a period of 6 months or longer.

Test Example

To a plastic plate to which cell nuclei had been fixed was added human serum obtained from normal persons (37 cases) or patients with autoimmune diseases (71 cases) in 1:100-diluted specimen in an amount of 40 $\mu$l per well. The human serum was diluted with a diluent prepared by dissolving casein in 0.01M Tris-HCl/0.15M NaCl buffer solution (pH 7.4, containing 0.1% $NaN_3$ as an antimicrobial agent) until saturated. After the addition of the diluted serum specimen, the plate was allowed to stand at room temperature for approximately one hour, followed by removal of unreacted supernatant from each well by suction. The wells were washed three times with a washing solution (an aqueous solution diluted, just before use, 20-fold from the stock solution containing 1.3 mg/ml of $Na_2HPO_4$, 3.3 mg/ml of $KH_2PO_4$, 100 mg/ml of NaCl and 20 mg/ml of monolaurate polyoxyethylene sorbitan). Then, 40 $\mu$l of a labelling solution prepared by dissolving HRPO-labelled protein G in a casein solution (the same as the diluent except for the use of 0.2% phenol as an antimicrobial agent) in a concentration of 5 mg/ml was added to each well. The protein G is a protein specifically reacting with immunoglobulin which is available from Cosmo Bio K.K. The wells were allowed to stand at room temperature for approximately one hour, followed by removal of the unreacted labelling solution from each well by suction and re-washing of each well with the washing solution.

To each well was then added 50 $\mu$l of a color-developing solution of tetramethylbenzidine (TMB) (an aqueous solution mixed, just before use, chromogen aqueous solution and substrate aqueous solution at the ratio of 1 to 10. Chromogen aqueous solution contains 5 mg/ml of tetramethylbenzidine, 0.2 mg/ml of phenoxymethylpenicillin pottasium and 3.2 $\mu$l/ml of hydrochloride. Substrate aqueous solution contains 0.27 mg/ml of urea-hydrogen peroxide, 13.1 mg/ml of sodium hydroxide and 1.64 $\mu$l/ml of acetic acid). After allowing to stand at room temperature for 30 min., the reaction was terminated by adding 50 $\mu$l of 0.5N sulfuric acid, and absurbance at 450 nm was measured.

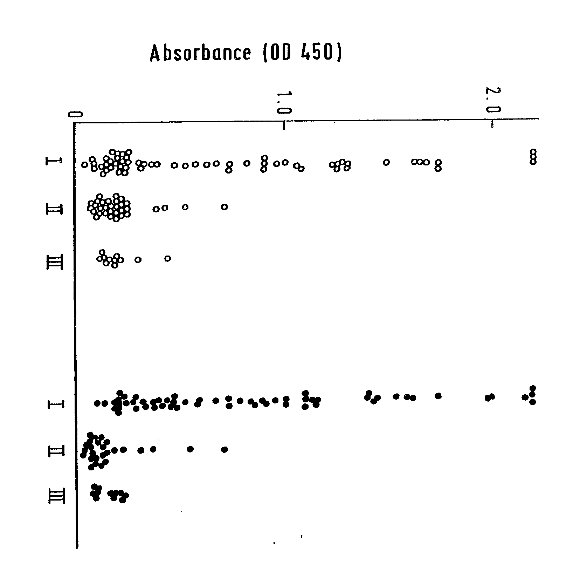

Results are shown in the attached drawing. In the figure, one circle open or solid represents one specimen. The open circle indicates the absorbance for each specimen in the case where the prior-art ELISA method using solubilized antigen (ENA) was carried out as control and the solid circle indicates that in the case where the device of the invention was employed. (I) is the case where measurement was made for the serum of patients with autoimmune diseases that had been positive by the fluorescent antibody technique using a glass slide with HEp-2 cells fixed which was a standard method heretofore. (II) is the case where normal human serum was measured, and (III) is the case where measurement was made for the serum from patients with autoimmune diseases that had been negative by the fluorescent antibody technique. The open circles most frequently found in the low-absorbance region indicate poor sensitivity of the prior-art device.

Whereas there was observed a correlation coefficient of 85-95% between the positive or negative judgement according to the ELISA using the nucleus fraction-carrying plate of the invention and the judgement according to the fluorescent antibody technique, there was observed a lower correlation coefficient of 60-70% in judgement between the prior-art ELISA using ENA and the fluorescent antibody technique. Further, a high correlation between the value measured by ELISA of the present invention and the intensity of fluorescence-staining was also observed.

Brief Description of the Drawing

The figures indicates results of the measurements of anti-nuclear antibody in serum specimens using the device according to the invention (open circles) and a prior-art technique (filled circles), respectively.

Claims

1) A method of preparing a device for measuring an anti-nuclear antibody which comprises contacting a suspension of cell nuclei with a support to adsorb the cell nuclei on the support, separating the support from said suspension and treating the surface of the support with a 50-90% acetone solution to fix the cell nuclei on the support.

2) A method according to claim 1 wherein a suspension of cell nuclei is poured to wells of a plastic support, followed by standing at room temperature for a period from 30 to 120 min. to adsorb the cell nuclei on the plastic support, removing unadsorbed supernatant from the wells, pouring to the wells a 50-90% acetone solution, which is allowed to stand at room temperature for a period of 10 min. or longer to fix the cell nuclei on the support.

3) A method according to claim 2 wherein cells washed with an isotonic solution are suspended in a hypotonic solution prepared in such that a salt concentration is in the range of 1/2 to 1/15 of that of the isotonic solution,
the cells are crushed in a homogenizer to a proportion of 90% or more,
nucleus fractions obtained by centrifugal separation of the crushed cell-containing solution are resuspended in said hypotonic solution,
the suspension of the nucleus fractions is poured to wells of a plastic support followed by standing at room temperature for a period from 30 to 120 min. to adsorb the nucleus fractions on the plastic support,
unadsorbed supernatant is removed from the wells, a 50-90% acetone solution is poured to the wells followed by standing at room temperature for a period of 10 min. or longer to fix the nucleus fractions on the plastic support,
the aceton solution is removed from the wells, and
the wells are dried in air.

4) A device for measuring an anti-nuclear antibody prepared according to any of claims 1 to 3.

5) The use of a device according to any of claims 1 to 3, for detection and/or determination of anti-nuclear antibodies.